# EUROPEAN PATENT SPECIFICATION

(11) **EP 1 872 730 B1**
(45) Date of publication and mention of the grant of the patent: **04.05.2011**
(21) Application number: 06729286.2
(22) Date of filing: 16.03.2006
(51) Int. Cl.: A61B 17/28, A61B 1/00

(54) **ENDOSCOPIC TREATMENT INSTRUMENT**
BEHANDLUNGSINSTRUMENT FÜR EIN ENDOSKOP
INSTRUMENT DE TRAITEMENT POUR ENDOSCOPE

(30) Priority: 21.04.2005 JP 2005123472
(43) Date of publication of application: 02.01.2008
(73) Proprietor: OLYMPUS MEDICAL SYSTEMS CORP., Tokyo 151-0072 (JP)
(72) Inventor: TAKAHASHI, Ichiro, 1910054 (JP)
(74) Representative: von Hellfeld, Axel
(86) International application number: PCT/JP2006/305293
(87) International publication number: WO 2006/114952

(56) References cited:
- WO-A-2005/060841
- JP-A- 10 506 032
- JP-A- 62 144 643
- US-A- 4 271 845

## Description

### TECHNICAL FIELD

The present invention is an endoscopic treatment instrument which is inserted into a channel of an endoscope so as to perform a treatment with respect to a living body.

Priority is claimed on Japanese Patent Application No. 2005-123472, filed April 21, 2005, the content of which is incorporated herein by reference.

### BACKGROUND ART

An endoscopic treatment instrument has a flexible insertion portion which can be inserted into a channel of an endoscope. The insertion portion is formed of a closely-wound coil, for example. An operation wire is inserted into the coil so as to freely advance and retreat. The operation wire is fixed to an operator provided in a proximal end (base end of the hand side) of the insertion portion and is connected to a driving portion of a biopsy cup or the like of a treatment portion provided in a distal end (leading end) of the insertion portion. Therefore, when the operation wire is advanced and retreated by the operator, the driving portion of the treatment portion can be driven.

In this case, if the insertion portion has high flexibility, the insertion portion is easily inserted into the channel, when an endoscope insertion portion of the endoscope is curved. On the other hand, when the entire insertion portion is excessively flexible, it is difficult to insert the insertion portion into the channel of the endoscope, because the insertion portion has no waist. Further, the insertion portion easily buckles. Therefore, an endoscopic treatment instrument is being developed, in which the hardness of a portion of an insertion portion, or specifically, a distal end side of the insertion portion is reduced rather than that or the other portion thereof. As a method for manufacturing such an endoscopic treatment instrument, a method is known in which coils having different hardness are thermally bonded, or the outer diameter of a distal end portion is reduced by a cutting process. Further, when a coil of the insertion portion is wound, the coil at the distal end portion thereof may be wound by a relatively small preload tension such that the rigidity of the coil is reduced (for example, refer to Patent Document 1: PCT Japanese Translation Patent Publication No. 10-506032). In addition, a winding angle or tension on winding a wire rod of a coil is selected to reduce the adhesion of the wire rod such that the rigidity of the coil is reduced (refer to Patent Document 2: Japanese Unexamined Patent Application, First Publication No. 62-144643).
Patent Document 1: PCT Japanese Translation Patent Publication No. 10-506032
Patent Document 2: Japanese Unexamined Patent Application, First Publication No. 62-144643

### DISCLOSURE OF THE INVENTION

### Problem to be solved by the Invention

However, when the coils having different hardness are thermally bonded or the cutting process is performed, man hours are required. Therefore, the manufacturing cost increases. Further, when the cutting process is performed, chips are generated. Therefore, a process of cleaning the coil to remove the chips is needed. Therefore, it is difficult to enhance productivity.

Further, to change the tension when the wire rod is wound, a coil winder additionally needs a complex mechanism. Furthermore, when the tension is changed while the coil is wound, the coil cannot be manufactured at high speed.

An advantage of the present invention is that it provides an endoscopic treatment instrument in which an insertion portion having a flexible distal end portion is simply manufactured at a low cost.

### Means for Solving the Problem

According to a first aspect of the invention, an endoscopic treatment instrument includes an insertion portion that is inserted into a channel of an endoscope and has flexibility; a treatment portion that is provided in a distal end side of the insertion portion and performs treatment with respect to a living body; and an operator that is provided in a base end portion of the insertion portion and operates the treatment portion. The operator and a driving portion of the treatment portion are connected through an operation wire which is inserted into the insertion portion so as to freely advance and retreat, the insertion portion includes a coil obtained by winding a wire rod, the coil has a flexible portion and a hard portion, which have different hardness from each other due to heat treatment, and the flexible portion having relatively low hardness is provided in a distal end side of the coil from the hard portion.

In the endoscopic treatment instrument, since the hardness of the flexible portion is relatively reduced by the heat treatment, the flexible portion can be more easily curved than the hard portion. Therefore, even when the insertion portion is inserted into a portion of the channel which is easily curved at the distal end portion of the endoscope, the insertion portion can be deformed along the shape of the channel.

According to a second aspect of the invention, an endoscopic treatment instrument includes an insertion portion that is inserted into a channel of an endoscope
Patent Document 1: PCT Japanese Translation Patent Publication No. 10-506032
Patent Document 2: Japanese Unexamined Patent Application, First Publication No. 62-144643

US 4,271,845 relates to a device for bending a medical instrument inserted into the body cavity having a flexible cylindrical coil formed by winding an elastic thin wire. The flexible cylindrical coil has a coarsely wound portion at one end portion thereof, that is designed by changing configuration or material so that one side of the coarsely wound portion is different in cross sectional area from the other side which is diametrically opposite. When a pulling string directly or indirectly fastened to the coarsely wound portion of the flexible cylindrical coil is operated, the turns of said flexible cylindrical coil are brought closer to each other on the side of the coarsely wound portion greater in torsional strength to thereby bend the one end portion of the flexible cylindrical coil towards the side of the coarsely wound portion.

### DISCLOSURE OF THE INVENTION

### Problem to be solved by the Invention

However, when the coils having different hardness are thermally bonded or the cutting process is performed, man hours are required. Therefore, the manufacturing cost increases. Further, when the cutting process is performed, chips are generated. Therefore, a process of cleaning the coil to remove the chips is needed. Therefore, it is difficult to enhance productivity.

Further, to change the tension when the wire rod is wound, a coil winder additionally needs a complex mechanism. Furthermore, when the tension is changed while the coil is wound, the coil cannot be manufactured at high speed.

An advantage of the present invention is that it provides an endoscopic treatment instrument in which an insertion portion having a flexible distal end portion is simply manufactured at a low cost.

### Means for Solving the Problem

According to the invention, an endoscopic treatment instrument includes the features of the independent claim. Preferred embodiments of the endoscopic treatment instrument are defined by the dependent claims.

As the annealing process is performed on the distal end side of the coil, the hardness thereof is relatively reduced, thereby forming the flexible portion. The other portion of the coil becomes the hard portion of which the hardness is relatively high, because the annealing process is not performed.

### Effects of the Invention

According to the invention, since the flexible portion of which the hardness is relatively reduced by the heat treatment is provided in the distal end side of the coil, the insertion into the channel of the endoscope and the treatment are easily performed. Therefore, damage which the treatment may apply to the channel of the endoscope can be reduced. For example, the channel can be prevented from being scraped or perforated. Further, as the flexible portion is formed by the heat treatment, a complex mechanism does not need to be provided in the manufacturing apparatus of the coil. Furthermore, the winding time of the coil can be reduced, and the cutting process is not needed. Therefore, it is possible to enhance productivity.

### BRIEF DESCRIPTION OF THE DRAWINGS

FIG 1 is a diagram showing the construction of an endoscopic treatment instrument according to an embodiment of the invention.
FIG. 2 is a cross-sectional view taken along line A-A of FIG. 1.
FIG. 3 is a cross-sectional view showing another example of FIG. 2.
FIG. 4 is a cross-sectional view showing a further example of FIG. 2.
FIG 5 is a cross-sectional view of a distal end portion of a coil.
FIG. 6 is a diagram for explaining a method of using the endoscopic treatment instrument, showing a state where the endoscopic treatment instrument is inserted into an endoscope.
FIG 7 is a diagram for explaining a process of manufacturing the endoscopic treatment instrument.
FIG. 8 is a diagram viewed in a direction of line B of FIG. 7.
FIG. 9 is a diagram for explaining a method of measuring the hardness of the coil.
FIG. 10 is a diagram for explaining a method of measuring the hardness of the coil.
FIG 11 is a diagram showing an endoscopic treatment instrument according to another embodiment of the invention.
FIG 12 is a diagram for explaining a method of using the endoscopic treatment instrument.
FIG. 13 is a diagram showing an endoscopic treatment instrument according to a further embodiment of the invention.
FIG 14 is a diagram for explaining a method of using the endoscopic treatment instrument.
FIG 15 is a cross-sectional view showing a state where holes are formed in the distal end portion of a coil.
FIG. 16 is a diagram showing a process of forming a slit in the distal end portion of the coil.
FIG. 17 is a cross-sectional view showing a cup holding portion formed in the distal end portion of the coil.

### Reference Numerals

1,81: ENDOSCOPIC BIOLOGICAL TEST FORCEPS (ENDOSCOPIC TREATMENT INSTRUMENT)
2: INSERTION PORTION
3: OPERATOR
4, 82: TREATMENT PORTION
10: COIL
13, 14, 62: OPERATION WIRE
17, 18: BIOPSY CUPS (DRIVING PORTION)
31: FLEXIBLE PORTION
32: HARD PORTION
40: ENDOSCOPE
43: CHANNEL
51: ANNEALING DEVICE
61: BASKET-TYPE FORCEPS (ENDOSCOPIC TREATMENT INSTRUMENT)
63: TREATMENT PORTION (DRIVING PORTION)

### BEST MODE FOR CARRYING OUT THE INVENTION

Hereinafter, an endoscopic treatment instrument according to an embodiment of the present invention will be described with reference to the drawings. The endoscopic treatment instrument according to the invention is described as an endoscopic biological test forceps, but is not limited thereto.

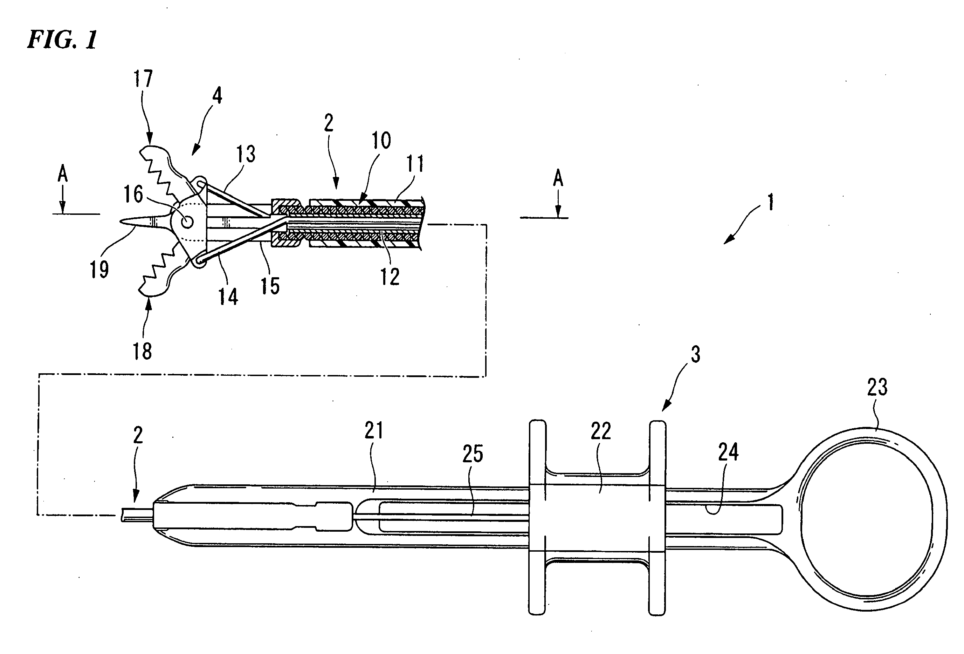

As shown in FIG. 1, an endoscopic biological test forceps 1 has a long insertion portion 2. The insertion portion 2 has an operator 3 provided in a proximal end (base end) thereof and a treatment portion 4 provided in a distal end (leading end) thereof. The operator 3 is used outside the body, and the treatment portion 4 is used for performing a treatment in the body.

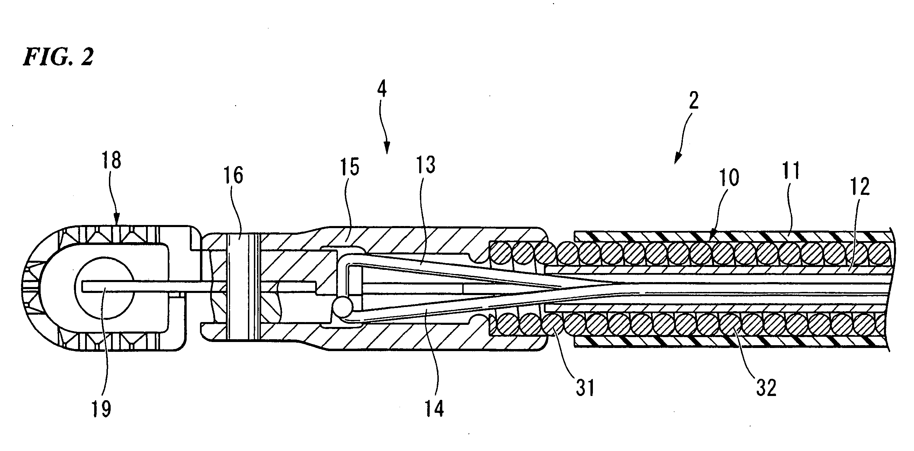

As shown in FIG. 2, the insertion portion 2 has a sheath formed on the outer surface of a closely-wound coil 10. The sheath is coated with an outer tube 11 formed of a heat-shrink tube through tube molding. The coil 10 has an inner tube 12 inserted therein such that the inner tube 12 freely advances and retreats. Further, the inner tube 12 has two operation wires 13 and 14 inserted therein such that the operation wires 13 and 14 freely advance and retreat.

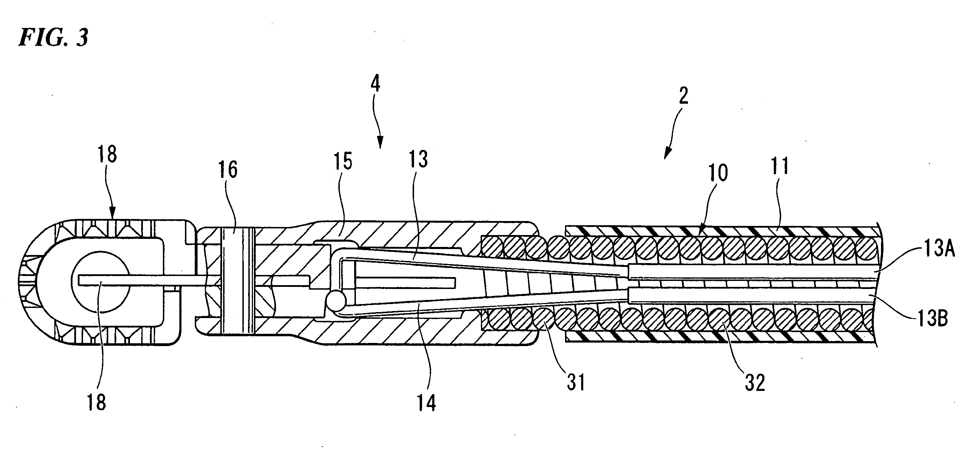

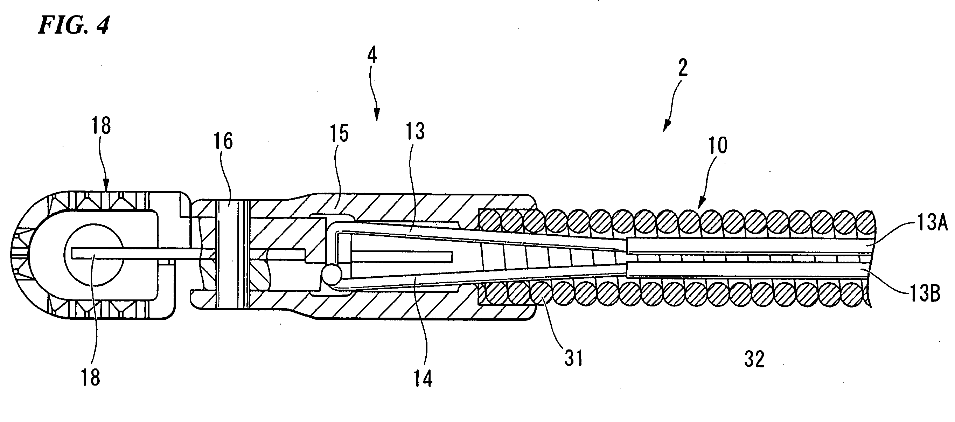

As shown in FIG. 3, the respective operation wires 13 and 14 may be coated with coating materials 13A and 14A, respectively, instead of the inner tube 12. The coating materials 13A and 14A are formed of resin such as high-density polyethylene or low-density polyethylene or a material obtained by mixing the high-density polyethylene and the low-density polyethylene. Further, as shown in FIG. 4, the coil 10 may be exposed without the outer tube 11 being provided.

As for the material of the outer tube 11, high-density polyethylene, a mixture of high-density polyethylene and low-density polyethylene, or a mixture of low-density polyethylene and polypropylene may be used, which has an excellent insertion property with respect to a channel of an endoscope at a low cost. Further, the outer tube 11 may be formed in a shape where unevenness on the surface of the coil 10 is transferred onto the outer surface of the outer tube 11, or the outer surface of the outer tube 11 may have an uneven shape in an axial direction thereof.

Further, the coil 10 has a cup holding member 15 fitted into a base end portion thereof, and the cup holding member 15 is fixed by laser welding, brazing, soldering, or caulking. Around a distal end portion of the cup holding member 15, a pin 16 is inserted in such a manner that a pair of biopsy cups (driving portions) 17 and 18 are rotatably supported by the pin 16. The operation wires 13 and 14 are connected to the base end portions of the biopsy cups 17 and 18, respectively. As the operation wires 13 and 14 are advanced and retreated, the pair of biopsy cups 17 and 18 can be opened and closed. Further, between the pair of biopsy cups 17 and 18, a needle 19 is disposed. The needle 19 is fixed to the cup holding member 15, and a sharp distal end of the needle 19 is formed to project from the cup holding member 15.

Meanwhile, the operator 13 has a slider 22 mounted on an operator body 21 in such a manner that the slider 22 freely advances and retreats. The insertion portion 2 is inserted into the operator body 21 from the distal end thereof, and a finger hooking ring 23 is provided in the base end of the operator body 21. Further, the operator body 21 has a groove 24 formed from the vicinity of the ring 23 toward the distal end portion thereof, and the slider 22 is mounted in the groove 24. The slider 22 has the base end portions of the operation wires 13 and 14 fixed thereto. When the slider 22 is moved along the groove 24, the operation wires 13 and 14 can be advanced and retreated. Between the distal end of the groove 24 and the slider 22, a rigid operation pipe 25 is inserted. As the operation wires 13 and 14 are inserted into the operation pipe 25, the operation wires 13 and 14 are prevented from buckling when the slider 22 is moved.

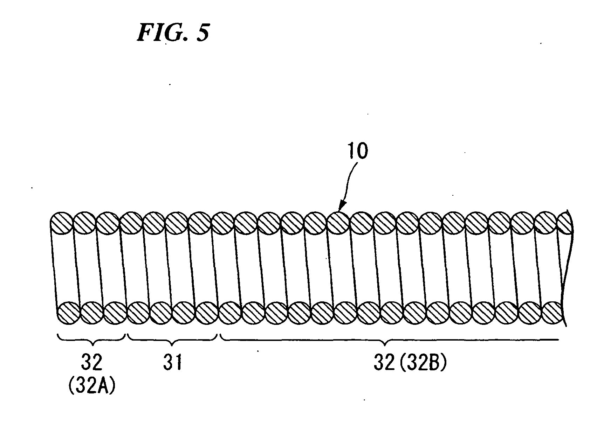

As shown in FIG. 5, the coil 10 has a flexible portion 31 formed in the distal end side thereof, the flexible portion 31 being a low-hardness portion. The flexible portion 31 has a lower hardness than the other portion of the coil 10, and the hardness is reduced by heat treatment without changing the outer diameter of the coil 10. The flexible portion 31 has a length of 2 to 30 mm from a portion where the flexible portion 31 is exposed from the cup holding member 15. Although the coil 10 has a hard portion 32A formed in the distal end side thereof from the flexible portion 31, the cup hold member 15 is attached to the hard portion 32A. Further, the coil 10 has a hard portion 32B formed in the base end side thereof from the flexible portion 31, the hard portion 32B being connected to the operator 3 shown in FIG. 1. The hard portion 32B is composed of a high-hardness portion. Further, without the hard portion 32A provided in the distal end side of the coil 10, the coil 10 may be composed of the flexible portion 31 and the hard portion 32B formed in the base end side thereof.

The outer and inner tubes 11 and 12 are formed of a resin material. For example, they are formed of a resin material, such as polytetrafluoroethylene (PTFE), tetrafluoroethylene-perfluoroalkoxy-ethylene resin (PFA), tetrafluoroethylenehexafluoropropylene resin (FEP), polyethylene, polypropylene, polyethylene terephthalate, ethylene vinyl acetate copolymer, polyolefin, polyamide, vinyl chloride, latex, or natural rubber, or a mixture of these resin materials.

Further, the coil 10, the operation wires 13 and 14, and the needle 19 are formed of a metallic material. For example, they are formed of metal, such as stainless steel, aluminum, nickel, brass, titanium, steel, phosphor bronze, tungsten, gold, silver, copper or the like, or an alloy of these metals.

The operator body 21 and the slider 22 can be thrown away (discarded) after use. Therefore, the operator body 21 and the slider 22 can be formed of a cheap resin material. For example, they are formed of a resin material, such as polyolefin, polycarbonate, acrylonitrile-butadiene-styrene resin, polyamide, vinyl chloride, latex, polypropylene, polyethylene terephthalate, ethylene vinyl acetate copolymer, natural rubber or the like, a mixture of these resin materials, or cross-linked resin obtained by irradiating electron beams onto these resin materials.

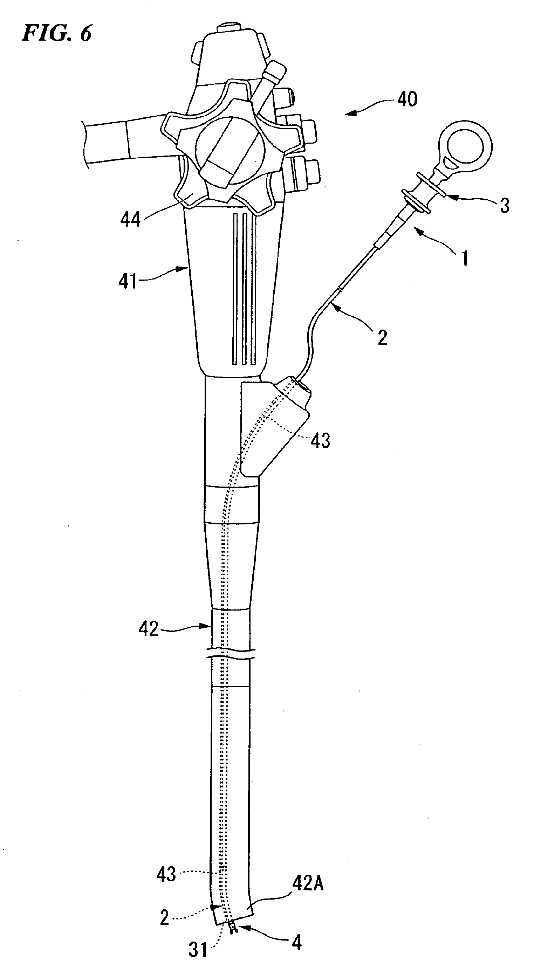

Now, an endoscope which is used together with the endoscopic biological test forceps 1 will be described with reference to FIG. 6. An endoscope 40 has an endoscope operator 41, which is operated by a person outside the body, and a flexible and long endoscope insertion portion 42 which extends from the endoscope operator 41, the endoscopic insertion portion 42 being inserted into the body. The endoscope insertion portion 42 has an imaging unit provided on the distal end thereof, the imaging unit being used for observing the inside of the body. Further, the endoscope insertion portion 42 has a distal end opening of a channel 43 formed in the distal end thereof. The channel 43 is connected to the endoscope operator 41 through the endoscope insertion portion 42 and has an insertion hole formed in a side of the endoscope operator 41. Inside the endoscope insertion portion 42, an angle wire (not shown) is inserted. When an angle knob 44 of the endoscope operator 41 is rotated, a distal end portion 42A can be curved.

Next, a method of manufacturing the endoscopic biological test forceps 1 will be described, while being focused on a process of manufacturing the distal end portion of the coil 10.

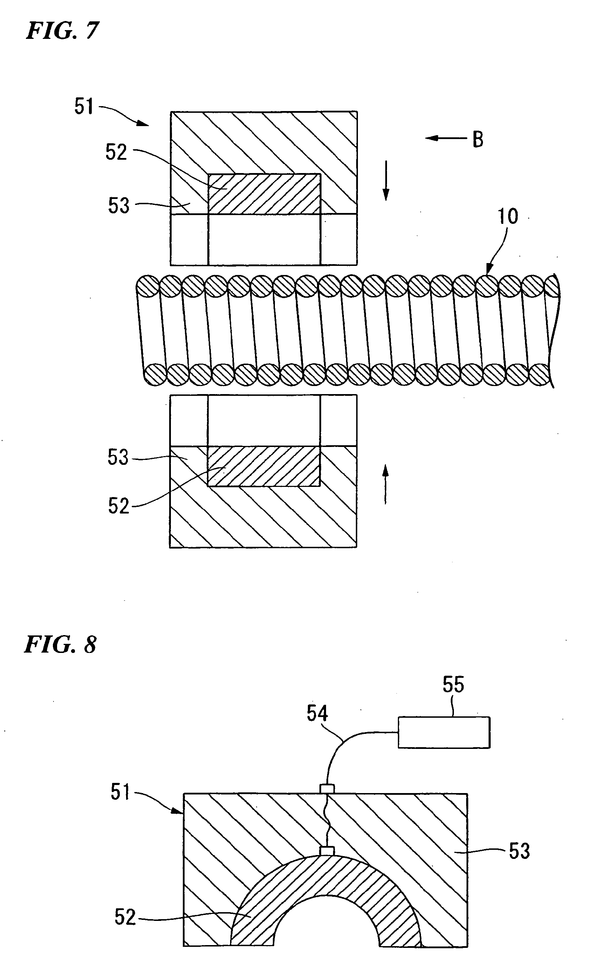

First, a wire rod is wound with a predetermined diameter by a winder (not shown) so as to manufacture a coil 10. Then, the distal end portion of the coil 10 is inserted into an annealing device 51, shown in FIGS. 7 and 8, so as to form the flexible portion 31. The annealing device 51 has a pair of high-frequency heaters 52 fixed by a pair of supporting portions 53, the high-frequency heaters 52 being formed in such a concave shape as to interpose the coil 10. The length of each of the high-frequency heaters 52 in a direction parallel to the axial direction of the coil 10 is substantially equal to that of the above-described flexible portion 31. For example, the length of the high-frequency heater 52 is set to 2 to 30 mm. The high-frequency heaters 52 are connected to a high-frequency power supply 55 through a cable 54.

When the coil 10 is interposed by the pair of high-frequency heaters 52, power is supplied to the high-frequency heaters 52 so as to heat the coil 10. In this case, the heating temperature is set to 500 to 8000, and retention time is set to 2 to 20 seconds. After the retention time passes, the power supply to the high-frequency heaters 52 is stopped, and simultaneously, the annealing device 51 is opened so that the heated portion of the coil is cooled. Then, the portion of the coil 10 interposed between the high-frequency heaters 52 is annealed and becomes the flexible portion 31 of which the hardness is relatively reduced. Since the other portion of the coil 10 is not heated, the hardness thereof does not change. Therefore, the other portion becomes the hard portion 32 of which the hardness is higher than that of the flexible portion 31.

Here, the hardness of the coil 10 having the flexible portion 31 formed therein will be described.

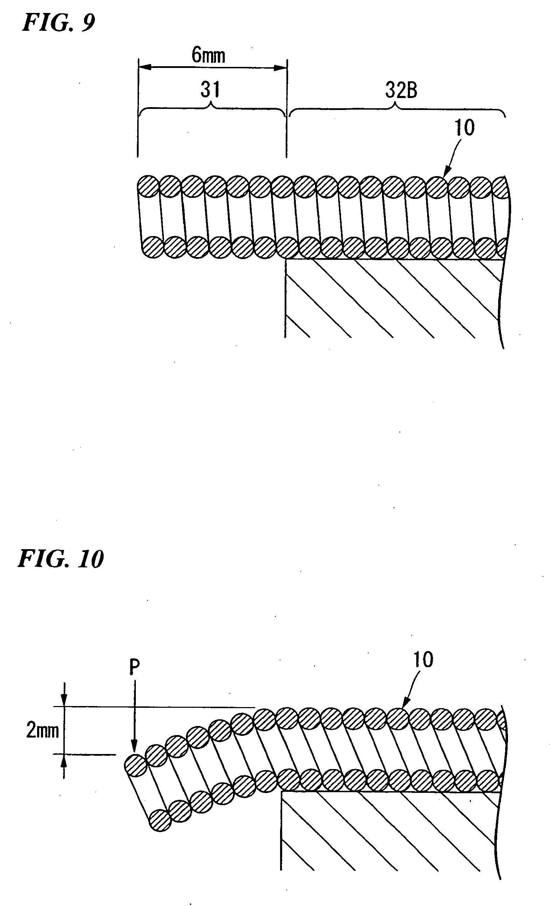

First, as shown in FIG. 9, the coil 10 having the flexible portion 31 formed in the distal end thereof is set to be horizontal. In this state, the base end of the coil 10 is fixed in such a manner that the distal end of the coil 10 is set to a cantilever composed of the flexible portion 31 with an overall length of 6 mm, for example. Then, as shown in FIG. 10, a downward load is applied on the distal end of the coil 10, and power P required for displacing the distal end of the coil 10 by 2 mm in a downward direction is measured before and after the annealing is performed. In the coil 10 after the annealing, required power P is reduced more than 10%, which means that the hardness of the flexible portion 31 is reduced by an amount corresponding to the reduction in power P.

As such, after the annealing is performed on the coil 10 so as to form the flexible portion 31, the endoscopic biological test forceps 1 is assembled. First, the coil 10 is coated with the outer tube 11, the inner tube 12 is inserted into the coil 10, and the operation wires 13 and 14 are inserted into the inner tube 12. Further, the treatment portion 4 is fixed to the distal end of the coil 10. Meanwhile, the distal end portion of the coil 10 is inserted and fixed to the operator body 21 of the operator 3. Then, the operation wires 13 and 14 are connected to the pair of biopsy cups 17 and 18 and the slider 22, respectively, such that the biopsy cups 17 and 18 are supported by the cup holding member 15.

When treatment is performed by the endoscopic biological test forceps 1, the endoscope 40 is first inserted into the body. At this time, the endoscope insertion portion 42 is curved along the shape of the gullet or the like. Further, the angle knob 44 of the endoscope operator 41 is operated in such a manner that the distal end portion 42A of the endoscope insertion portion 42 is curved toward a treatment target portion so as to check the treatment target portion through an imaging unit. The channel 43 is curved along the endoscope insertion portion 42. In particular, the distal end portion 42A of the endoscope insertion portion 42 can be curved further than the channel 43. When the treatment target portion is checked, the endoscopic biological test forceps 1 is inserted into the insertion hole of the endoscope operator 41 in such a manner that the treatment portion 4 is first inserted. The insertion portion 2, which is connected to the treatment portion 4 so as to be inserted into the channel 43, is introduced into the endoscope insertion portion 42 while being curved along the channel 43. At a position where the insertion portion is largely curved at the distal end of the channel 43, the flexible portion 31 is curved more largely than the other portion (the hard portion 32B) of the coil 10. Therefore, the treatment portion 4 easily passes through the position so as to project from the distal end portion of the endoscope insertion portion 42.

In a state where the treatment portion 4 projects toward the treatment target portion, the slider 22 of the operator 3 is operated so as to advance the operation wires 13 and 14 fixed to the slider 22. When the operation wires 13 and 14 are relatively advanced with respect to the coil 10, the pair of biopsy cups 17 and 18 connected to the distal ends of the operation wires 13 and 14, respectively, are opened. Then, after the biopsy cups 17 and 18 are pressed against the treatment target portion, the slider 22 is retreated. Further, the pair of biopsy cups 17 and 18 are opened so as to interpose the treatment target portion. In this state, when the insertion portion 2 is pulled toward the outside of the body, the treatment target portion is taken.

According to this embodiment, since the flexible portion 31 which is more flexible than the other portion is formed adjacent to the hard treatment portion 4, the endoscopic biological test forceps 1 is easily deformed along the shape of the channel 43. Therefore, damage which the treatment 4 may apply to the channel 43 of the endoscope 40 can be reduced. For example, the channel 43 can be suppressed from being scraped or perforated. Further, in the coil 10, the flexible portion 31 is provided only in the vicinity of the treatment portion 4, and the shape of the coil 10 is not changed in the flexible portion 31 and the hard portion 32. Therefore, when the operation wires 13 and 14 are advanced, or when the entire endoscopic biological test forceps 1 is advanced, high compression resistance is obtained. Therefore, while the flexibility is secured, the operationality may be enhanced.

In addition, since the flexible portion 31 is formed by performing the annealing on a portion of the coil 10, a chipping process such as a conventional cutting process or a cleaning process is not needed. Therefore, it is possible to manufacture the endoscopic biological test forceps at a low cost and within a short time. Further, since a special mechanism does not need to be provided in the winder of the coil 10, it is possible to reduce the manufacturing cost. Furthermore, if the annealing is performed on a plurality of coils 10 at a time, it is possible to enhance productivity.

Next, a second embodiment of the invention will be described in detail with reference to the drawings. Like reference numerals are attached to the same components as those of the first embodiment, and the duplicated descriptions thereof will be omitted.

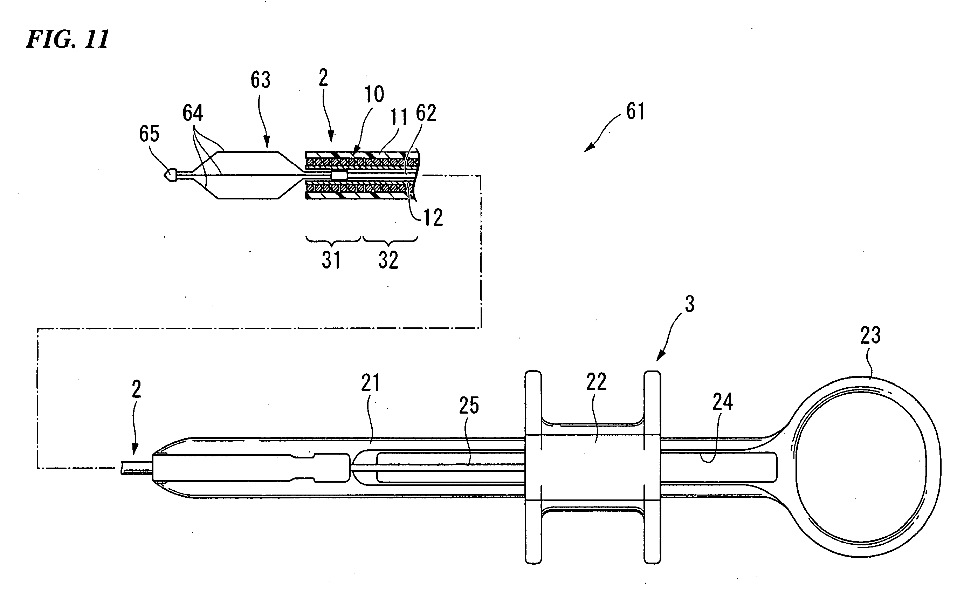

In this embodiment, an endoscopic treatment instrument is a basket-type forceps 61, as shown in FIG. 11. In the basket-type forceps 61, an operation wire 62 is inserted into the inner tube 12 of the insertion portion 2 so as to freely advance and retreat, and a base end portion of the operation wire 62 is fixed to the slider 22 of the operator 3. Further, the operation wire 62 has a treatment portion 63 attached to the distal end portion thereof. The treatment portion 63 has a plurality of wire rods 64 fixed to the operation wires 62. The wire rods 64 are respectively deformed in a widening direction, and the distal ends of the wire rods 64 are tied by a chip 65. The wire rods 64 are formed of an elastically-deformable material. When the operation wire 62 is pulled, the wire rods 64 are housed into the inner tube 12. When the operation wire 62 is pushed, the wire rods 64 are projected from the insertion portion 2 so as to be widely opened.

The coil 10 has a flexible portion 31 formed in the range of 20 to 300 mm from the distal end surface thereof. The other portion of the coil 10 is composed of a hard portion 32. The manufacturing direction and hardness of the flexible portion 31 are the same as those of the first embodiment.

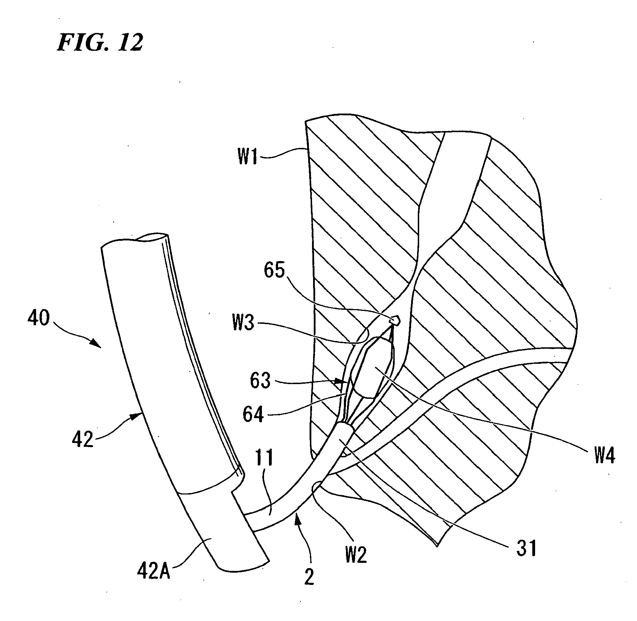

When the basket-type forceps 61 is used, it is inserted into the channel 43 of the endoscope 40. When the distal end portion of the insertion portion 2 passes through the curved portion of the distal end portion 42A of the endoscope insertion portion 42, the flexible portion 31 of the coil 10 is deformed along the shape of the channel 43. Further, even when the basket-type forceps 61 is projected from the distal end portion 42A of the endoscope insertion portion 42 so as to perform treatment, the flexible portion 31 is deformed so that the treatment is easily performed. For example, as shown in FIG 12, when the insertion portion 2 of the basket-type forceps 61 is inserted into a bile duct W3 from a papilla W2 of the duodenum W1 such that a bilestone W4 is destroyed by the treatment portion 63, the flexible portion 31 of the coil 10 is easily curved so that the insertion portion 2 is easily inserted into the bile duct W3 from the papilla W2.

According to this embodiment, since the flexible portion 31, which is easily deformed, is provided in the distal end side of the insertion portion 2, the insertion or intrusion into a living body is easily performed. Therefore, an operation is easily performed, and operation time can be reduced. The other effects are the same as those of the first embodiment.

Next, a third embodiment of the invention will be described in detail with reference to the drawings. In this embodiment, the endoscopic treatment instrument is described as an endoscopic biological test forceps. Like reference numerals are attached to the same components as those of the first embodiment, and the descriptions thereof will be omitted.

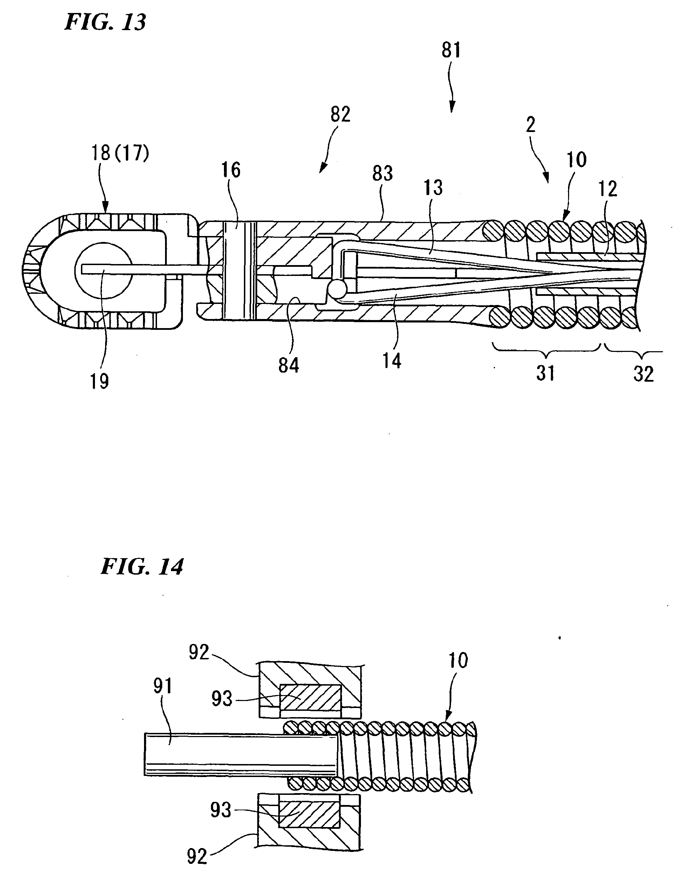

As shown in FIG. 13, an endoscopic biological test forceps 81 has the insertion portion 2. The coil 10 serving as a constituent component of the insertion portion 2 has a cup holding portion 83 of a treatment portion 82 integrally formed in the distal end thereof. The cup holding portion 83 has a slit 84 extending in a longitudinal direction from the distal end surface thereof and a pin 16 fixed to cross the slit 84 at right angles. The pin 16 supports a pair of biopsy cups 17 and 18 such that the biopsy cups 17 and 18 can be freely opened and closed. The function of the cup holding portion 83 is the same as that of the cup holding member 15 of the first embodiment.

A process of manufacturing the cup holding portion 83 will be described with reference to FIGS. 14 to 17.

First, a cored bar 91 having an outer diameter substantially equal to the inner diameter of the coil 10 is inserted into the coil 10 from the distal end side of the coil 10, and is then interposed by a pair of molds 92. On the opposed surfaces of the respective molds 92, a pair of substantially semi-circular concave portions are formed. A pair of high-frequency heaters 93 are provided in the concave portions, respectively. The high-frequency heaters 93 have an inner diameter smaller than the outer diameter of the coil 10. Therefore, when the molds 92 are clamped while the coil 10 is heated by the high-frequency heaters 93, the wire rods of the distal end portion of the coil 10 are deformed into a cylindrical shape by hot forging while being melted. When the molds 92 are opened after a predetermined time passes, a cylindrical portion is obtained, in which the wire rods adjacent to each other at the distal end portion of the coil 10 are thermally bonded and connected to each other. At this time, the coil 10 connected to the cylindrical portion is not deformed, but receives an effect caused by the heat. Then, as the coil 10 is slowly cooled, the hardness thereof decreases. Therefore, the vicinity of the cylindrical portion becomes the flexible portion 31.

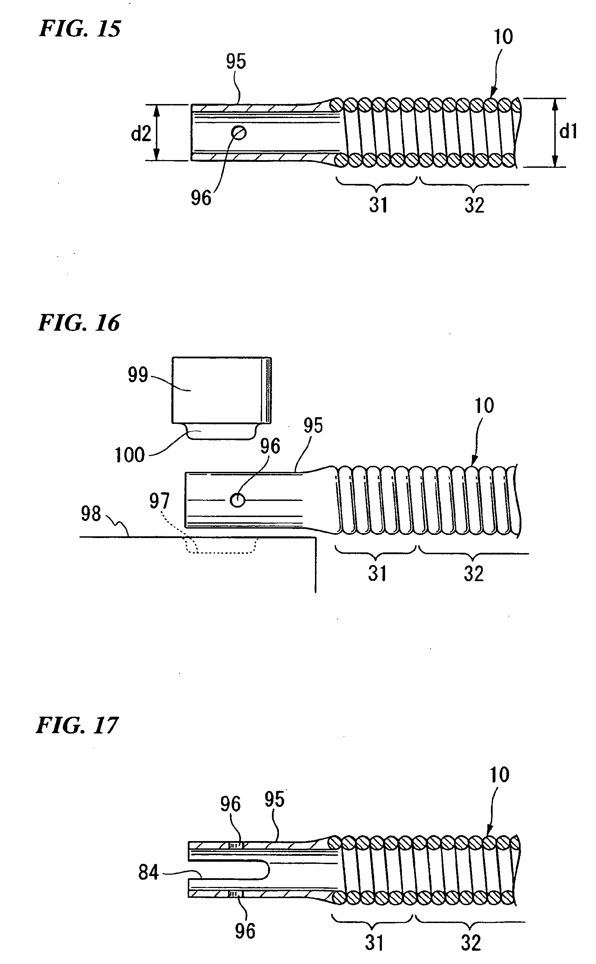

Next, as shown in FIG. 15, after the cylindrical portion 95, having an outer diameter d2 smaller than the outer diameter d1 of a portion which is not molded by hot forging, is molded, a pair of holes 96 passing through the cylindrical portion 95 is formed at the distal end of the coil 10. The holes 96 are formed in a position where they are axially symmetrical with respect to the cylindrical portion 95. The holes 96 are formed by the following method. The cylindrical portion 95 is punched from the upper side thereof by a punch so as to form the holes 96. In this case, a cored bar having a through-hole formed in accordance with the formation position of the holes 96 may be inserted into the cylindrical portion 95, without punching the cylindrical portion 95.

In addition, as shown in FIG. 16, the cylindrical portion 95 is loaded on a lower mold 98 having a concave portion 97 formed therein such that the axis line of the hole 96 becomes horizontal. Then, the cylindrical portion 95 is punched by an upper mold 98 from the upper side. Since the upper mold 99 has a projecting portion 100 which is fitted into the concave portion 97 of the lower mold 98, a slit 84 is formed in the cylindrical portion 95 by the projecting portion 100. As a result, the cup holding portion 83 is formed in the distal end side of the coil 10, as shown in FIG 17. After that, when the pair of biopsy cups 17 and 18 connected to the operation wires 13 and 14, respectively, are supported by the pin 16, the treatment portion 82 is formed.

According to this embodiment, the cup holding portion 83 of the treatment portion 82 is integrally formed by heat-treating the distal end of the coil 10. Therefore, the number of parts can be reduced, and cost reduction can be realized. Further, when the cup holding portion 83 is formed, the flexible portion 31 can be formed in the coil 10 connected to the cup holding portion 83. Therefore, the manufacturing process can be simplified. The effect caused by the formation of the flexible portion 31 is the same as that of the first embodiment.

The present invention is not limited to the above-described embodiments, but can be widely applied.

For example, in the third embodiment, when the flexible portion 31 is formed, the same annealing device 51 as that of the first embodiment may be disposed in parallel to the pair of molds 92 such that the flexible portion 31 is formed by the annealing device 51.

Various methods of manufacturing the flexible portion 31 can be adopted. For example, the distal end side of the coil 10 is heated by a burner or the like, and is then cooled. Further, without annealing the distal end of the coil 10, the other portion of the coil 10 excluding the distal end portion thereof may be heat-hardened so that the flexible portion 31 is relatively formed.

Further, the invention can be applied to an insertion portion of another endoscopic treatment instrument such as a snare, a high-frequency knife or the like.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

## Claims

1. An endoscopic treatment instrument (1) comprising:
an insertion portion (2) that is insertable into a channel (43) of an endoscope and has flexibility;
a treatment portion (4) that is provided in a distal end side of the insertion portion and is adapted to perform treatment with respect to a living body; and
an operator (3) that is provided in a base end portion of the insertion portion and is adapted to operate the treatment portion;
wherein the operator and a driving portion of the treatment portion are connected through an operation wire, which is inserted into the insertion portion so as to freely advance and retreat,
the insertion portion includes a coil (10) obtained by winding a wire rod,
the coil has a flexible portion (31) and a hard portion (32), which have different hardness from each other due to heat treatment,
the flexible portion having relatively low hardness is provided in a distal end side of the coil from the hard portion, **characterized in that**
a shape of the coil is not changed in the flexible portion and the hard portion,
the endoscopic treatment instrument comprises a rigid operation pipe (25) into which the operation wire (13, 14) is inserted so as to freely advance and retreat, and the operator has a slider (22) mounted on an operator body (21) in such a manner that the slider can freely advance an retreat, wherein the operator body has a groove (24) formed from the base end of the operator body to the distal end of the operator body, the slider is mounted in the groove and the operation pipe is inserted between the distal end of the groove and the slider.

2. An endoscopic treatment instrument according to claim 1, wherein the hardness of a distal end portion of the coil is reduced rather than that of the other portion of the coil, such that a flexible portion is formed.

3. The endoscopic treatment instrument according to claim 1 or 2, wherein the flexible portion is formed by an annealing process.

## Patentansprüche

1. Ein endoskopisches Behandlungsinstrument (1), aufweisend:
einen Einführabschnitt (2), der in einen Kanal eines Endoskops einführbar ist und Flexibilität hat;
einen Behandlungsabschnitt (4), der an einer distalen Endseite des Einführabschnitts angeordnet ist und eine Behandlung an einem lebenden Körper durchzuführen vermag; und
einen Betätiger (3), der an einem Basisendabschnitt des Einführabschnitts angeordnet ist und den Behandlungsabschnitt zu betätigen vermag;
wobei der Betätiger und ein Antriebsabschnitt des Behandlungsabschnitts über einen Betätigungsdraht verbunden sind, der in den Einführabschnitt frei vor- und zurückbeweglich eingesetzt ist,
der Einführabschnitt eine Schraubenwicklung (10) enthält, die durch Wickeln eines Drahtstabs erhalten wird,
die Schraubenwicklung einen flexiblen Abschnitt (31) und einen harten Abschnitt (32) aufweist, welche aufgrund einer Wärmebehandlung zueinander unterschiedliche Härten haben,
der flexible Abschnitt mit relativ niedriger Härte in einer distalen Endseite der Schraubenwicklung vom harten Abschnitt her angeordnet ist, **dadurch gekennzeichnet, dass**
sich eine Form der Schraubenwicklung in dem flexiblen Abschnitt und dem harten Abschnitt nicht ändert,
das endoskopische Behandlungsinstrument eine steife Betätigungsröhre (25) aufweist, in welche der Betätigungsdraht (13, 14) frei vor- und zurückbeweglich eingesetzt ist, und
der Betätiger einen Gleiter (22) aufweist, der an einem Betätigerkörper (21) derart angebracht ist, dass der Gleiter frei vor- und zurückbeweglich ist, wobei der Betätigerkörper eine vom Basisende des Betätigerkörpers aus zum distalen Ende des Betätigerkörpers hin ausgebildete Ausnehmung (24) aufweist, wobei der Gleiter in der Ausnehmung angeordnet ist und die Betätigungsröhre (25) zwischen das distale Ende der Ausnehmung und den Gleiter eingesetzt ist.

2. Das endoskopische Behandlungsinstrument nach Anspruch 1, wobei die Härte eines distalen Endabschnitts der Schraubenwicklung mehr als diejenige des anderen Abschnitts der Schraubenwicklung verringert ist, so dass ein flexibler Abschnitt gebildet ist.

3. Das endoskopische Behandlungsinstrument nach Anspruch 1 oder 2, wobei der flexible Abschnitt durch einen Temperprozess gebildet ist.

## Revendications

1. Instrument de traitement endoscopique (1) comprenant :
une partie d'insertion (2) qui est insérable dans un canal (43) d'un endoscope et a une flexibilité ;
une partie de traitement (4) qui est prévue dans un côté d'extrémité distale de la partie d'insertion et est adaptée à exécuter un traitement pour ce qui concerne un corps vivant ; et
un opérateur (3) qui est prévu dans une partie d'extrémité de base de la partie d'insertion et est adapté à faire fonctionner la partie de traitement ;
dans lequel l'opérateur et une partie d'entraînement de la partie de traitement sont connectés par l'intermédiaire d'un fil opérationnel qui est inséré dans la partie d'insertion de manière à avancer et reculer librement,
la partie d'insertion inclut une bobine (10) obtenue en enroulant un fil machine,
la bobine a une partie flexible (31) et une partie rigide (32) qui ont une dureté différente l'une par rapport à l'autre du fait d'un traitement thermique,
la partie flexible ayant une dureté relativement faible est prévue dans un côté d'extrémité distale de la bobine par rapport à la partie rigide, **caractérisé en ce que**
une forme de la bobine n'est pas changée dans la partie flexible et la partie rigide,
l'instrument de traitement endoscopique comprend un tuyau opérationnel (25) rigide dans lequel le fil opérationnel (13, 14) est inséré de manière à avancer et reculer librement, et
l'opérateur a un curseur (22) monté sur un corps (21) d'opérateur de telle manière que le curseur peut avancer et reculer librement, dans lequel le corps d'opérateur a une rainure (24) formée de l'extrémité de base du corps d'opérateur jusqu'à l'extrémité distale du corps d'opérateur, le curseur est monté dans la rainure et le tuyau opérationnel est inséré entre l'extrémité distale de la rainure et le curseur.

2. Instrument de traitement endoscopique selon la revendication 1, dans lequel le dureté d'une partie d'extrémité distale de la bobine est réduite plutôt que celle de l'autre partie de la bobine, de telle manière qu'une partie flexible est formée.

3. Instrument de traitement endoscopique selon la revendication 1 ou 2, dans lequel la partie flexible est formée par un procédé de recuit.
